# EUROPEAN PATENT APPLICATION

(11) **EP 3 485 931 A1**
(43) Date of publication of application: **22.05.2019**
(21) Application number: 17202933.2
(22) Date of filing: 21.11.2017
(51) Int. Cl.: A61M 25/00

(54) **URETERAL CATHETER**

(71) Applicant: Sana-one GmbH, 38820 Halberstadt (DE)
(72) Inventor: Ehlert, Valerie, 83043 Bad Aibling (DE); Maier, Florian, 83022 Rosenheim (DE)
(74) Representative: Habermann, Hruschka & Schnabel

(57) **Abstract**

The present invention relates to a ureteral catheter suitable for use as a guide catheter in the ureter making wire guides for introducing of the catheter into the ureter dispensable. The ureteral catheter of the invention has a flexible tip section at the distal end and, proximally to the tip section a shat section being provided with anti-kink stability. The shaft section has a multilayer composition comprising an outer layer having a hydrophyilic outer surface, a medium layer proving the anti-kink stability, and an inner layer having an inner surface showing low friction for metallic surfaces. The catheter of the present invention is also useful for guiding a ureteral stent.

## Description

The present invention relates to a ureteral catheter suitable for use as a guide catheter in the ureter making wire guides for introducing and pushing the catheter into the ureter dispensable. The ureteral catheter of the invention has a flexible tip section at the distal end and, proximally to the tip section, a shaft section being provided with anti-kink stability. At least the shaft section has a multilayer composition comprising an outer layer having a hydrophyilic outer surface, a medium layer proving the anti-kink stability, and an inner layer having an inner surface showing low friction for metallic surfaces. The catheter of the present invention is also useful for guiding a ureteral stent into the ureteral tract.

Ureteral soft-body catheters of the prior art have commonly to be introduced into the ureteral system by use of a guide wire. Other catheters of the prior art have a more rigid body and comprise a hydrophilic coating. However, the latter rigid catheters are not easily introduced into the ureteral tract.

CN 204050612 (U) discloses a ureteral catheter having a catheter body made by the aid of variable-strength reinforcing mesh weaving technique. The surface of the catheter body has a hydrophilic coating for enhancing the lubricity of the surface of the catheter body.

EP 2 258 435 A1 discloses a urinary catheter having a low-friction surface by treatment of with a liquid swelling medium prior to use of the catheter.

CN 204601339 (U) describes a disposable soft ureteral catheter assembly comprising a soft multicavity pipe into which a push rod is inserted for pushing the assembly into the ureteral duct.

The technical problem underlying the present invention is to provide a ureteral catheter that can be introduced into the ureteral system without the need of a guide wire, and which catheter can be used as guide catheter for ureteral stents.

The solution of the above technical problem is provided by the embodiments of the present invention as described herein and defined in the claims.

In particular, the present invention provides a ureteral catheter comprising distally a flexible tip section at the distal end and proximally a non-kinking shaft section wherein at least the shaft section comprises at least a segment having a multilayer assembly comprising
(i) an inner layer with an inner surface having a low friction coefficient for metallic surfaces;
(ii) a medium layer providing stability against kinking; and
(iii) an outer layer having an outer surface supporting the introduction and pushing of the catheter in the ureteral tract.

A "catheter" according to the invention is, generally speaking, an elongate tubular device having a proximal end and a distal end and a passageway defining an inner lumen extending between those ends.

In one embodiment of the invention, especially in case the catheter of the invention is used as a guide catheter, the tip has a closed configuration. In other embodiments of the tip section has at least an opening for discharging liquid from the catheter, The one or more opening(s) may be provided centrally at the distal end of the tip section. Alternatively, the opening(s) is (are) positioned laterally, in particular at the distal end of the tip section.

Considering ease of introducing of the catheter according to the invention into the urinary tract and pushing the catheter forward without damaging the tissue of the ureteral tract, the tip section has preferably a round shaped distal end.

The catheter according to the invention comprises at least two sections of different flexibility: the tip section which typically has a length varying from about 30 to about 100 mm, more preferably from about 50 to about 80 mm, most preferred it has a length of about 70 mm, is located at the distal end of the catheter tube and is relatively flexible in comparison to the proximal shaft section which is - at least in a region or section thereof - resistant against kinking.

The anti-kinking properties may be provided by means generally known in the art: the material of the medium layer may be a non-kinking material such as a non-kinking plastic material. In one embodiment the medium layer, at least of the shaft section, comprises at least a section comprising a fabric enforcing said section of the medium layer. The fabric may be selected from a wide variety of materials such as textiles, metals, alloys or other known materials. The stiffness of the fabric-enforced medium layer can be varied by the fabric used for enforcing the medium layer and/or the type braiding or coiling of the fabric fibres in the medium layer, by different modes of winding of the fabric around the axis of the shaft. The fibres or wires of the fabric may be round or flat. The fibres may be arranged in a braid fashion wherein the characteristic parameter of the density of the braid is given as the PIC count (no. of fibre or wire, respectively, crosses per inch). Another type of arrangement of the fibres or wires, respectively, in fabric enforcement is a coil whereby the density of the coiling is given as WPI count (no. of wraps per inch). A higher PIC or WPI count, respectively, increases the flexibility, while fabrics with a lower PIC or WPI count, respectively, increase the longitudinal stiffness of the material.

According to a preferred embodiment of a fabric-enforced medium layer, the fabric reinforcing the medium layer (ii) is positioned in the boundary area of the medium layer towards the outer layer (iii).

The materials used for providing the various layers of the present catheter are typically chosen from various commercially available plastic materials. Corresponding polymers, their mixtures or blends thereof, include polyethylene (PE), polyvinylchloride (PVC), polycarbonate (PC), polyamide (PA), polyurethane (PUR), particularly thermoplastic polyurethane (TPU), polysiloxane, polytetrafluorethylene (PTFE), thermoplastic polyether-block-amide (PEBA), polyether-etherketone (PEEK) and polyetherimide (PEI).

For the flexible tip of the invention, softer materials are typically chosen such as PEBA. The shaft section has typically a medium layer of a stiffer material such as PEI, PEEK, TPU or PA which may additionally be reinforced by fabrics as described above.

Alternatively, the tip section may be provided with more flexibility than the shaft section by ablation of material from the medium layer in the tip section so that said medium layer has less thickness at least in part of the tip section as compared to the shaft section. In one embodiment, the ablation may be constant over the tip section. In other embodiments, ablation may be carried out differently in different parts of the tip section, and may be carried out to provide regular or other patterns of ablative regions in the tip section.

The inner layer (i) is preferably selected from lubricous polymer materials such as fluoro or perfluoro, respectively, polymers, preferably PTFE, PVDF (polyvinylidene fluoride) or FEP (fluorinated ethylene-propylene).

A particularly preferred embodiment of the invention is a catheter having distally a flexible tip section and a proximal shaft section wherein both sections comprise a multilayer assembly comprising (i) an inner layer of PTFE, (ii) a fabric-reinforced medium layer which is of PEBA in the tip section and of PEI in the shaft section, and (iii) an outer hydrophilic layer.

The catheter according to any one of the preceding claims wherein further embedding layers are provided between layers (i) and (ii) and/or (ii) and (iii).

The catheter according to the invention typically has a total length of about 1500 to about 1800 mm.

The catheter has preferably inner diameter of at least about 0.65 mm.

For being suited as a guide catheter for placement of ureteral stents, the outer diameter of the catheter is preferably at most about 0.96 mm and ranges preferably from about 0.89 to about 0.96 mm.

For supporting the introduction of the catheter of the present invention into the ureteral tract and also supporting it at pushing forward, the outer surface of layer (iii) provides the catheter with slippage in respect of the tissue surfaces in the ureteral tract. In one preferred embodiment, the outer layer is made of a hydrophilic polymer and/or has a hydrophilic coating. Hydrophilic polymers for use as the outer layer are typically polymers having polar groups such as polyamides, polyimide and hexamethylene-diisocyanate-parylene. Hydrophilic coatings for ureteral catheters being suitable in the context of the invention include polyvinylpyrrolidone. Alternatively, the outer surface of the outer layer (iii) may be provided with other polymers or polymer coatings, respectively, providing the catheter with the required slippage in the ureteral tract. Certain hydrophobic polymers may be used for this purpose, and examples include outer layers made of, comprising or being coated with, respectively, a Parylene® such as Parylene® N, Parylene® C, Parylene® D or Parylene® HT.

In a further preferred embodiment, at least the shaft section of the catheter has a printable outer surface. In a preferred embodiment of this type, a length graduation can be printed onto the outer surface. Accordingly, the catheter of the invention comprises a length graduation, preferably in cm and/or inches, at least on part of the shaft section.

According to another preferred embodiment, the catheter of the invention is at least in part visible under x-rays such that manipulation and pushing of the catheter are facilitated at use under x-ray radiation. X-ray visibility can, for example, be provided by x-ray visible fibre materials, e.g. metals or alloys, reinforcing the medium layer in the shaft section according to the respective embodiments as outlined above. Alternatively, wire of metal or other x-ray visible materials such as alloys may be included into the catheter or a part thereof separately or additionally.

Further subject matter of the invention is a kit comprising the catheter according to any one of the preceding claims in combination with a ureteral stent. Ureteral stents are known in the art and available from various manufacturers. The catheter of the present invention is preferably combined with systems that previously required the use of quide wires of 0.035 or 0.038 inch (0,089 to 0.096 cm) diameter.

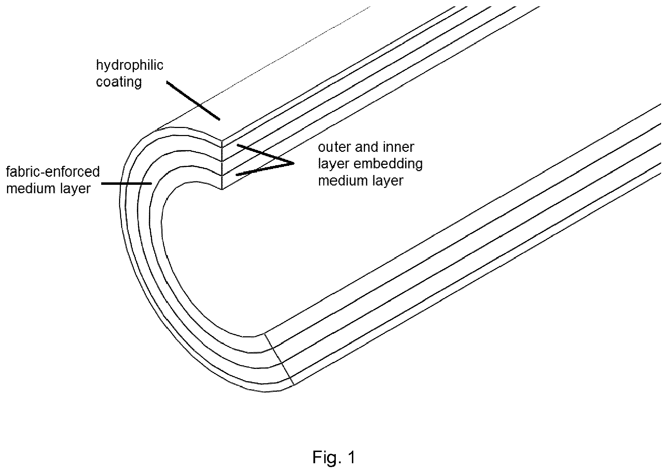

- Fig. 1: shows a perspective cross-sectional view demonstrating the multilayer structure of a catheter of the invention. A medium layer, here reinforced by fabric, is embedded between an inner lubricous layer and an outer layer having a hydrophilic coating.

## Claims

1. Ureteral catheter comprising distally a flexible tip section at the distal end and proximally a non-kinking shaft section wherein at least the shaft section comprises at least a segment having a multilayer assembly comprising
(i) an inner layer with an inner surface having a low friction coefficient for metallic surfaces;
(ii) a medium layer providing stability against kinking; and
(iii) an outer layer having an outer surface supporting the introduction and pushing of the catheter in the ureteral tract.

2. The catheter of claim 1 wherein the tip section has at least an opening for discharging liquid from the catheter.

3. The catheter of claim 2 wherein the opening(s) is (are) positioned centrally at the distal end of the tip section.

4. The catheter of claim 2 wherein the opening(s) is (are) positioned laterally at the distal end of the tip section.

5. The catheter according to any one of the preceding claims wherein the tip section has a round shaped distal end.

6. The catheter according to any one of the preceding claims wherein the medium layer comprises at least a section comprising a fabric enforcing said section of the medium layer and/or comprising a non-kinking plastic material.

7. The catheter of claim 6 wherein the fabric reinforcing the medium layer (ii) is positioned in the boundary area of the medium layer towards the outer layer (iii).

8. The catheter according to any one of claims 1 to wherein the medium layer comprises a polymer selected from the group consisting of polyamide, polyether-block-amide (PEBA TPA), thermoplastic polyurethane, polyether-etherketone (PEEK) and polyethylene imide (PEI) and mixtures thereof.

9. The catheter of claim 8 wherein the medium layer (ii) of the shaft section comprises PEI.

10. The catheter of claim 8 or 9 wherein the tip section comprises PEBA TPA.

11. The catheter according to any one of the preceding claims wherein the inner layer (i) comprises a fluoropolymer, preferably PTFE or FEP.

12. The catheter according to any one of the preceding claims having an outer surface providing slippage with respect to the surface of the tissue in the ureteral tract.

13. The catheter of claim 12 wherein the outer layer has a hydrophilic outer surface.

14. The catheter according to any one of the preceding claims wherein further embedding layers are provided between layers (i) and (ii) and/or (ii) and (iii).

15. The catheter according to any one of the preceding claims wherein the tip section makes up about 30 mm to about 100 mm of the total length of the catheter.

16. The catheter of claim 15 wherein the tip makes up about 70 mm of the total length of the catheter.

17. The catheter according to any one of the preceding claims having a total length of about 1500 to about 1800 mm.

18. The catheter according to any one of the preceding claims wherein the inner diameter of the catheter is at least about 0.65 mm.

19. The catheter according to any one of the preceding claims having a length graduation on at least a part of the shaft section.

20. The catheter according to any one of the preceding claims being at least in part visible under x-rays.

21. The catheter according to any one of the preceding claims wherein the outer diameter of the catheter is at most 0.96 mm, preferably from 0.89 to 0.96 mm.

22. The catheter according to any one of the preceding claims wherein the outer layer is made of a hydrophilic polymer and/or has a hydrophilic coating.

23. A kit comprising the catheter according to any one of the preceding claims in combination with a ureteral stent.
